# EUROPEAN PATENT APPLICATION

(11) **EP 4 306 204 A1**
(43) Date of publication of application: **17.01.2024**
(21) Application number: 22192405.3
(22) Date of filing: 26.08.2022
(51) Int. Cl.: B01D 53/48, C12P 7/6463, C12N 1/20, B01D 53/34, C12P 7/625, C12P 39/00

(54) **MICROBIOLOGICAL PROCESS FOR THE CONVERSION OF CARBON DIOXIDE INTO A BIOPRODUCT**

(30) Priority: 13.07.2022 US 202263368291 P; 26.07.2022 GB 202210917
(71) Applicant: CEMVITA FACTORY, INC., Houston, TX 77054 (US)
(72) Inventor: DA SILVA, Marcio Luis Busi, Houston, 77054 (US); GUPTA, Rajesh, Houston, 77054 (US); NELSON, Charles, Houston, 77054 (US); HENDERSON, John, Houston, 77054 (US); TAHER, Edris, Houston, 77054 (US); KINCAID, Kevin Patrick, Houston, 77054 (US)
(74) Representative: Brand Murray Fuller LLP

(57) **Abstract**

The present invention provides a combined process of bio-desulfurization and carbon capture utilising chemotrophs to produce one or more bio-products.

## Description

### TECHNICAL FIELD

The present invention relates to chemotrophic carbon capture and desulfurization and concerns a combined process of bio-desulfurization and carbon capture utilising chemotrophs to produce one or more bio-products.

Bio-desulfurization is a known process for removing sulfur/sulfides from gas streams (e.g. natural gas, associated gas, syngas, amine acid gas, flue gas, landfill gas, biogas, effluent from anaerobic digesters), and also from liquid streams such as diesel and crude oil. The primary or sole focus of conventional bio-desulfurization processes is the removal of sulfur (e.g. H₂S). Such processes are not typically concerned with carbon capture - either at all or with conditions desirable for its optimization. In fact, conventional bio-desulfurization processes such as the Thiopaq O&G Process generally seek to minimize the amount of biomass build-up (carbon capture) for ease of operation of the bio-desulfurization process.

### BACKGROUND TO THE INVENTION

US 11,091,781 and US 10,597,681 disclose systems and methods for fixing carbon using bacteria. The systems include a reaction chamber with a solution contained therein. The solution may include hydrogen, carbon dioxide, bioavailable nitrogen, and a chemolithoautotrophic bacteria. The system may also include a pair of electrodes that split water contained within the solution to form the hydrogen. Additionally, the system may be operated so that a concentration of the bioavailable nitrogen in the solution is below a threshold nitrogen concentration to cause the chemolithoautotrophic bacteria to produce a product.

US 2022/0154228 discloses compositions and methods for a hybrid biological and chemical process that captures and converts carbon dioxide and/or other forms of inorganic carbon and/or CI carbon sources and/or mixtures containing CI chemicals into organic chemicals including biofuels or other valuable biomass, chemical, industrial, or pharmaceutical products.

US 9,957,534 discloses microorganisms containing exogenous or heterologous nucleic acid sequences, wherein the microorganisms are capable of growing on gaseous carbon dioxide, gaseous hydrogen, syngas, or combinations thereof. In some embodiments the microorganisms are chemotrophic bacteria that produce or secrete at least 10% of lipid by weight. Also disclosed are methods of fixing gaseous carbon into organic carbon molecules useful for industrial processes, and methods of manufacturing chemicals or producing precursors to chemicals useful in fuels.

US 10,507,426 and US 9,764,279 disclose methods and systems to achieve clean fuel processing systems in which carbon dioxide emissions from sources may be processed in at least one processing reactor containing a plurality of chemoautotrophic bacteria which can convert the carbon dioxide emissions into biomass which may then be used for various products such as biofuels, fertilizer, or feedstocks. Sulfate-reducing bacteria may be used to supply sulfur containing compounds to the chemoautotrophic bacteria.

US 10,376,837 discloses methods and systems to achieve clean fuel processing systems in which carbon dioxide emissions from sources may be processed in at least one processing reactor containing a plurality of chemoautotrophic bacteria which can convert the carbon dioxide emissions into biomass which may then be used for various products such as biofuels, fertilizer, or feedstocks. Bacteria that reduce oxidized nitrogenous species may be used to supply reduced nitrogenous compounds to the chemoautotrophic bacteria.

US 10,543,458 discloses a process to treat a gas comprising hydrogen sulfide and mercaptans, comprising the steps: (a) contacting the hydrogen sulfide and mercaptans comprising gas with an aqueous solution comprising sulfide-oxidising bacteria thereby obtaining a loaded aqueous solution and a gas having a lower content of hydrogen sulfide and mercaptans, (b) contacting the loaded aqueous solution with mercaptan reducing microorganisms immobilized on a carrier under anaerobic conditions, (c) separating the aqueous solution obtained in step (b) from the mercaptan reducing microorganisms to obtain a first liquid effluent, and (d) contacting the first liquid effluent with an oxidant to regenerate the sulfide-oxidising bacteria to obtain a second liquid effluent comprising regenerated sulfide-oxidising bacteria. The sulfide-oxidising bacteria as present in step (a) are comprised of regenerated sulfide-oxidising bacteria obtained in step (d).

US 10,801,045 discloses pathways, mechanisms, systems and methods to confer chemoautotrophic production of carbon-based products, such as sugars, alcohols, chemicals, amino acids, polymers, fatty acids and their derivatives, hydrocarbons, isoprenoids, and intermediates thereof, in organisms such that these organisms efficiently convert inorganic carbon to organic carbon-based products of interest using inorganic energy, such as formate, and the use of organisms for the commercial production of various carbon-based products.

The prior art is primarily concerned with either removal of sulfur (e.g. H₂S) or CO₂ capture and/or utilisation. The prior art fails to provide a process where carbon capture is linked with sulfur removal.

In a process for producing one or more bio-products comprising of capturing carbon dioxide (CO₂), the removal of further impurities such as hydrogen sulfide (H₂S) results in the production of an improved product. None of the prior art documents contain any satisfactory teaching as to how to remove H₂S in combination with such a process where carbon is captured using a microorganism.

### SUMMARY OF THE INVENTION

The object of the present invention is to provide an improved process for capturing carbon dioxide to produce one or more bio-products, in which sulfur (e.g. H₂S) is also removed and utlilized, unlike in conventional processes in the art.

In this specification one or more bio-products means one or more products derived from organic material available on a renewable or recyclable basis, and may include materials, chemicals and energy. Specific examples of bio-products include, but are not limited to, biomass, biochemicals, lipids, carbohydrates, and alcohols, and include polyhydroxyalkanoates, polyhydroxybutyrates, polyhydroxyvalerates and the like.

A further object of the present invention is to provide a system for carrying out the improved process that is efficient when compared to conventional methods, that is capable of enhancing bio-product production, and that allows control of bio-product composition.

According to the present invention there is provided a microbiological process for the conversion of CO₂ into one or more bio-products, in which the energy required for the microbiological conversion is at least partially provided by the concomitant microbiological oxidation of a sulfurous feedstock.

The microbiological process is preferably effected by one or more chemotrophic organisms.

Preferably one or more of the chemotrophic organisms are sulfur oxidising bacteria.

Preferably the sulfurous feedstock is at least partially oxidized to elemental sulfur by the sulfur oxidising bacteria.

Chemotrophic organisms may be provided as a single genus, species and or strain. Consortia of different organisms may also be used. The organisms may be aerobic, anaerobic, facultatively or otherwise.

The invention finds utilization in any industrial process in which both sulfide (or sulfanyl - typically primarily H₂S but also mercaptans/thiols etc. may be envisaged) and CO₂ are present. The process of the invention can be applied in downstream facilities such as refineries and chemical plants, midstream facilities such as acid gas treatment plant and acid gas injection wells, and upstream industry such as sour oil and gas wells, biogas and landfill gas facilities. The operators of such facilities when utilising the process of the invention may benefit from regulatory/governmental incentives with respect to carbon sequestration, and also stand to achieve effective desulfurisation without resort to capital-intensive and operationally complex Claus units and processes.

Therefore the present invention provides a microbiological process in accordance with the foregoing comprising the steps of:
i. contacting raw carbon dioxide and sulfur-containing feedstocks, either separately or in combination, with an absorption or dissolution medium to form a reagent stream comprising dissolved or absorbed inorganic carbon and sulfur; and
ii. contacting at least a portion of the reagent stream with a microbial broth in a bioreactor to oxidize sulfur and produce one or more bio-products from the carbon dioxide.

The dissolved or absorbed inorganic carbon is typically provided in the form of a bicarbonate or carbonate (preferably at least mostly bicarbonate).

The dissolved or absorbed inorganic sulfur is typically provided in the form of a sulfide, sulfhydryl or sulfanyl (most commonly sulfhydryl - e.g. NaSH).

The process of the invention may make use of the pH control taught in our co-pending but currently unpublished application USSN 63364275 filed May 6, 2022 - the entire disclosure of which is hereby incorporated by reference.

The process of the invention may further comprise:
iii. separating the one or more bio-products into one or more bio-products and a liquid stream; and
iv. recycling at least a portion of the liquid stream to step i. of the process for use as, or part of, the absorption or dissolution medium

A key advantage of the present invention is combined CO₂ capture and sulfur removal in a single process which can both abate greenhouse gas emissions, as well as safely remediate hazardous sulfurous streams.

As is well known, carbon dioxide (CO₂) is a common greenhouse gas emission, produced by burning fuels. Conventional methods of carbon capture include photosynthetic approaches, and sequestration in oil deposits. Hydrogen sulfide (H₂S) is a highly hazardous gas, which is toxic to humans and inhibits cellular respiration. Conventionally H₂S is removed from fuel gases by amine gas treating technology, where it is converted to an ammonium salt, and subsequently to elemental sulfur.

Linking carbon capture with H₂S removal has a distinct advantage in that oxidation of H₂S to elemental sulfur provides the electrons and energy needed for carbon fixation, reducing the overall energy required for the process compared to conventional processes in the art, and in the process of the invention generating one or more useful bio-products.

The pH of the absorption or dissolution medium is preferably controlled to facilitate its capacity to absorb or dissolve carbon dioxide and to contribute to reaction conditions in the bioreactor conducive to production of the one or more bio-products, as is taught in our aforementioned co-pending but currently unpublished application USSN 63364275 filed May 6, 2022.

The required pH control may be effected by biological means (e.g. selection of suitable microbes and/or microbial digestion conditions), chemical means (e.g. by adjusting the pH of the absorption or dissolution medium or of the recycle stream which contributes to it), or by both.

The pH of the absorption or dissolution medium may for example be adjusted by the addition of make up water. Often it will be desirable to increase the pH of the recycle stream within a desired range, which can be achieved by the addition of alkaline make up water.

The recycle stream may have a pH of between about 10.5 and about 11.5. In one embodiment, the recycle stream has a pH of about 11.

Advantageously, a pH of between about 10.5 and about 11.5 prevents live bacteria from entering the gas contactor.

In some embodiments, make up water or solvent may be added to the recycle stream prior to the recycle stream returning to the gas contactor and/or bioreactor.

Preferably, the pH of the liquid stream from the gas contactor to bioreactor is maintained between 8 and 10, preferably between 9 and 10, most preferably between 9 and 9.5. In one embodiment, the pH of the recycle stream is between 9 and 9.5.

If the pH of the liquid stream is not within the desired range (for example, between 9 and 10, most preferably between 9 and 9.5), an alkaline compound, for example, sodium hydroxide, may be added into makeup water to maintain the pH in the desired range. Therefore, in some embodiments the makeup water comprises an alkaline compound. In some further embodiments, the makeup water comprises sodium hydroxide.

The alkaline compound, for example sodium hydroxide, may be added to the makeup water to increase the pH of the makeup water to between about 12 and 13. Preferably, the resulting pH of the makeup water is about 12.5.

Increasing the pH of the makeup water can helps sterilize the recycle stream. Sterilization of the recycle stream can further be achieved by any methods known in the art, including, but not limited to, raising the pH of the recycle stream to prevent growth, adding biocide, adding bleaching agent(s), UV sterilization, gamma irradiation or increasing temperature.

Additionally, increasing the pH may improve carbon dioxide absorption and prevent unwanted microbial growth in the gas contactor. It may also help avoid sulfuric acid formation in the bioreactor.

The process of the invention is particularly effective in reducing the energy required for carbon capture and utilisation, and removing harmful contaminants from the bio-products (for example biomass used to produce fuels). In conventional chemical processes a large part of energy required for carbon capture is consumed in regenerating the solvent used for the CO₂ and H₂S absorption. In the present invention, biological systems are used to consume the dissolved CO₂ present in the form of bicarbonate in solution, and the H₂S present in the form of elemental sulfur, thereby producing the recyclable solvent and saving the energy conventionally needed for regeneration of solvent.

The source of the fluid feedstock stream may for example be flue gas, ambient air, and/or any other process gas stream(s) containing carbon dioxide and sulfurous material (such as hydrogen sulfide) - the raw feedstock(s). The use of such sources advantageously aids in reducing otherwise detrimental carbon dioxide and sulfurous emissions. The raw feedstock may be provided as a single stream comprising a mixture of CO₂ and sulfurous material (e.g. H₂S), or as two separate streams individually comprising CO₂ and sulfurous material (e.g. H₂S).

The contacting of the raw feedstock(s) with an absorption or dissolution medium to form a reagent stream comprising dissolved inorganic carbon and sulfur may be effected in a suitable vessel such as a gas contactor (or, as will be explained below, more than one gas contactor). The absorption/dissolution medium typically has a relatively low carbon dioxide and sulfurous content, which is substantially increased by contact with the raw feedstock. Thus, the process may further comprise increasing the carbonaceous and sulfurous content of the recycle stream by dissolving or absorbing CO₂ and sulfurous feedstock into the reagent stream, for example with the aid of one or more gas contactors.

As mentioned, the process may utilize two (or more) separate gas contactors, especially when there are separate streams of CO₂ and sulfurous feedstock to the process. A system comprising two (or more) gas contactors provides a greater degree of operational flexibility - for example by making it easier to control the C/S ratio in the reagent stream. The C/S ratio may be important in controlling the specification of the end bio-product(s). When the sulfurous stream is H₂S in that case w/w CO₂/H₂S ratio in the (combined) feedstock may be from about 0.1:1 to about 100:1, for example from about 1:1 to about 50:1, preferably from about 2:1 to about 25:1, more preferably from about 3:1 to about 15:1, for example about 6 or 7:1, in particular about 6.5:1.

The C/S ratio may also be expressed in molar terms (and it will be noted that H₂S is not necessarily the sulfurous feedstock). The molar ratio C/S is preferably around 10:1 to about 0.1:1, for instance from about 5:1 to about 0.5:1. A molar ratio of approximate parity (i.e. about 1:1) may sometimes be desirable.

The ratio of C/S can have important consequences in the process of the invention - for example when the bioreactor is charged with a consortia of microbiological organisms, the selection of C/S ratio will cause some of those organisms to respond preferentially, thereby impacting the nature of the end product. For example, when the end product is biomass - and it is desired to produce biomass with high lipid content - the C/S ratio may be selected accordingly to favour those microorganisms which yield the desired biomass compositional characteristic.

There are various types of commercially available gas contactors known in the art. The gas contactor suitable for use in the present invention may be dependent on the composition of the inlet fluid stream. Known commercially available contactors my include, but are not limited to, absorption columns with unstructured packing, absorption columns with structured packing or absorption columns with trays.

The packing of the gas contactors or trays utilized are specifically designs to improve gas and/or liquid mass transfer. Such designs are known and can be found, for example from Perry's Chemical Engineers' Handbook. New York: McGraw-Hill, 1984*.*

The raw feedstock may be pre-supplied to such a gas contactor before entering the bioreactor. The gas contactor and the bioreactor may therefore be provided as separate zones. Alternatively, the gas contactor and bioreactor may be provided as a single hybrid zone. In this case, it will be understood that in the process of the present invention the gas contactor and the bioreactor may be a single vessel, for example a tubular plug flow reactor. In this case, the recycle stream would be fed directly into the bioreactor in addition to the fluid stream comprising carbon dioxide and hydrogen sulfide.

The raw feedstock may be treated within the gas contactor to produce a carbon-rich feedstock liquid (the reagent stream comprising dissolved or absorbed inorganic carbon) and an off-gas substantially free of carbon dioxide. For example the gas contactor may be configured to absorb carbon dioxide from the raw feedstock (for example, ambient air, flue gas or any other process gas stream containing CO₂) to produce a concentrated carbon liquid stream (as reagent stream) and an off-gas substantially free of carbon dioxide.

The off-gas substantially free from carbon dioxide may be utilized in any commonly known process. For example, the off-gas free from carbon dioxide may be used in processes such as hot air regeneration of adsorbents or used as a compressed air feed.

The raw feedstock may optionally be fed through a pressure booster prior to entering the gas contactor and/or the bioreactor. Optionally the raw feedstock may be pre-treated prior to entering the process, for example by clean-up or concentration.

The raw carbonaceous feedstock may comprise up to about 100% v/v carbon dioxide, but may comprise various other materials, such as are typically found in industrial effluents, flue gases, off-gases and/or ambient air.

The raw sulfurous feedstock may comprise up to about 100% v/v H₂S, but may comprise various other materials, such as are typically found in industrial effluents, flue gases and/or off-gases.

The amount of carbon dioxide may be balanced mainly by nitrogen, water and oxygen. Additional trace amounts of SOₓ, NOₓ, H₂S, particulate matter, CO and other compounds typically present in industrial gas, petrochemical, or other heavy industrial operations may be present.

The microbial broth comprises a solvent selected from at least one of amine, alkaline solution (for example, sodium hydroxide or potassium hydroxide), using wastewater for bioremediation of organic stream, ammonia and/or enzyme (for example, carbonic anhydrase) .

The inventors have found that such an environment in the bioreactor provides a controlled environment that achieves optimal microbial growth.

The sulfur oxidizing bacteria (SOB) can be found in prior art descriptions. These include, but are not limited to, *Thioalkalimicrobium, Thioalkalivibrio, Thiobacillus, Alkalilimnicola, Guyparkeria, Halomonas, Alkalispirillum, Vibrio, Thiomicrospira, Guyparkeria, Thioalkalispira* (formerly *Thioalkalimicrobium), Ectothiorhodospiraceae, Rhodobacteraceae, Roseinatrobacter, Alkalilimnicola, Guyparkeria, Desulfuromusa, Desulfurispirillum.*

The bio-product composition or conversion efficiency may be optionally improved through genetic engineering and enhanced reactor design.

The biological means used in the present invention may be dependent on the final bio-product.

The bioreactor conditions may be controlled through in-line monitoring to determine the C/S ratio and carbon speciation. This information can be used to optimize the operating pH and gas flow rates to maximize CO₂ capture and system performance.

The nutrient supply to the process of the invention is controlled to maximise bio-product formation. Preferably the nutrient supply to the bioreactor is above 2mg(N)/l and/or 2mg(P)/l, preferably above 2.5, more preferably above 3 mg(N or P)/l, and is manipulated to maximise the amount of bio-product (e.g. biomass) produced. This nutrient concentration is typically higher than is conventional in current bio-desulfurisation technology - in fact, in such conventional technology the nutrient supply is deliberately maintained below these ranges.

The process of the invention yields one or more bio-products, such as biomass, PHA/B, 1,4-butanediol, amongst others, with desirably high lipid, carbohydrate and/or protein contents. In one aspect, the process may be adapted to preferentially to produce lipids in preference to protein and carbohydrates. This may be achieved by operating the bioreactor in a time-limited nutrient starved environment (i.e. with low N or P bioavailability) in order temporarily to stress the microorganisms and encourage lipid production.

It may also be desirable to limit the amount of oxygen supplied to the bioreactor in order to avoid the formation of sulfur dioxide. Running the bioreactor "oxygen-lean" in this way helps maximise elemental sulfur production by the sulfur oxidising bacteria.

Advantageously, the process according to the present invention can be used to produce biomass with different characteristics.

When the one or more bio-products comprises biomass, it may in some circumstances be desirable to maximise lipid content - in which case the biomass may comprise at least about 40%w/w lipid content, for example at least about 50%w/w lipid content, e.g. 60%w/w or 70%w/w lipid content.

Alternatively, where for example PHA/PHB or BDO is targeted as the end product the lipid content may be lower - for example 40%w/w or less, for instance 30%w/w or less.

The one or more bio-products produced from the process according to the present invention may be used, by way of non-limiting example, as an energy source, a final product, an intermediate product in a different process, or a carbon storage medium. Preferable end uses may be dependent on the product composition. In the instance that the one or more bio-products is biomass with a high lipid content will be desirable for biofuel production, such as biodiesel. Conversely, high carbohydrate content is more favourable for production of ethanol, and high protein biomass is suited for the production of protein supplements for agricultural feed or nutraceuticals.

In one embodiment, there may be a method of separating the reduced elemental sulfur from biomass. Depending on the location and end product, this may include centrifugation, solvent extraction, hydro cyclone, or any other separation method known in the art.

In another embodiment, once the sulfur is extracted and the target molecules removed from the one or more bio-products, there will be a portion of waste biomass or biomass debris. This debris can be used as a feed into anaerobic digester for biogas generation. The debris can also be utilized to feed a secondary bioreactor. The secondary bioreactor can optionally be a reactor with sulfate reducing bacteria (SRBs) which may produce H₂S to be fed back into the first reactor to improve carbon capture. This will be useful where sites are lacking sufficient H₂S for complete carbon capture. This second reactor may also be specifically designed to produce a number of other biologic chemicals, such as PHA/B, 1,4-butanediol, or other.

According to a second aspect of the present invention, there is provided an apparatus for the conversion of CO₂ into one or more bio-products, in which the energy required for the microbiological conversion is at least partially provided by the concomitant microbiological oxidation of a sulfurous feedstock, the apparatus comprising:
i. means for contacting raw carbon dioxide and sulfur-containing feedstocks, either separately or in combination, with an absorption or dissolution medium to form a reagent stream comprising dissolved or absorbed inorganic carbon and sulfur; and
ii. means for contacting at least a portion of the reagent stream with a microbial broth in a bioreactor to oxidize sulfur and produce one or more bio-products from the carbon dioxide.

An apparatus according to the present invention may further comprise a gas contactor to concentrate the fluid stream with high carbon content, and/or a second bioreactor for producing H₂S to be recycled and improve carbon capture.

The process according to the present invention may optionally further comprise at least one downstream process.

The downstream process may comprise a process that produces a gas comprising carbon dioxide, for example a biofuels production process.

The downstream process may comprise a process that converts elemental sulfur into a useful end product such as a fertiliser or commodity chemical.

By-products or products from the downstream process may advantageously be recycled and used in the process according to the present invention, thereby improving the environmental impact of the overall process and reducing the need for the introduction of external means.

### FIGURES AND EXAMPLES

The invention will now be more particularly described with reference to the following examples and Figures, in which;
Figure 1 depicts a schematic diagram of a process of sequestering carbon and removing H₂S according to the invention, wherein a co-mingled feed is used.
Figure 2 depicts a schematic diagram of a process of sequestering carbon and removing H₂S according to the invention, wherein separate feeds are used.
Figure 3 depicts a schematic diagram of a process according to the invention in which elemental sulfur produced by the process is recycled via a sulfur reducing bioreactor to generate H₂S for use in the process as the sulfurous feedstock.

### Figure 1

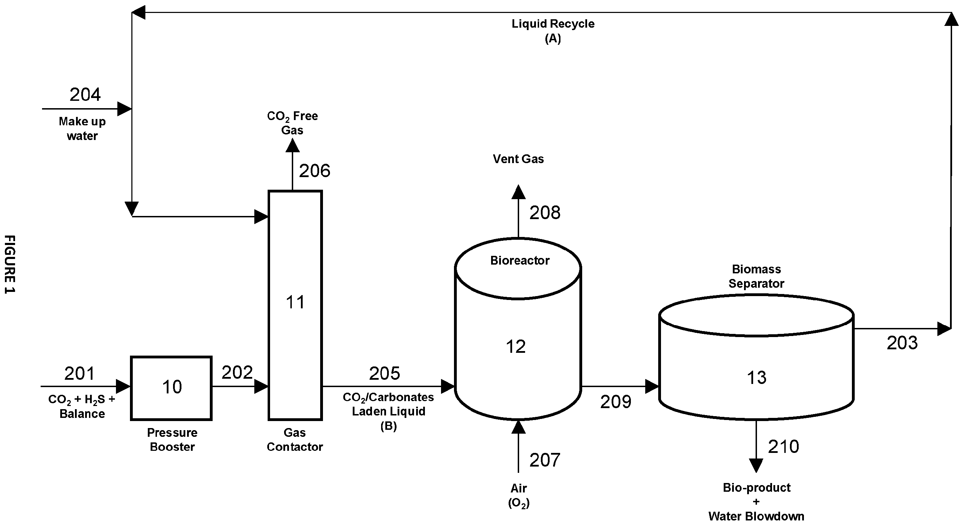

In the process of the invention schematically represented by Figure 1 the feedstock gas comprising CO₂ and H₂S is supplied in line 201 at atmospheric pressure to a pressure booster 10 which increases the pressure of the feedstock gas to an extent so the flue gas overcomes hydraulic head in gas contactor 11. Pressurized gas passes through line 202 to gas contactor 11, which is also supplied by line 203 with a liquid recycle stream from the process, in combination with make-up water from line 204. In gas contactor 11, CO₂ and H₂S in the feedstock stream is dissolved in the liquid recycle stream and is supplied on in line 205 to bioreactor 12. A substantially carbon dioxide free and hydrogen sulfide free off-gas is vented in line 206.

Stream B in line 205 enters bioreactor 12 charged with a microbial broth and maintained under conditions of temperature/pressure/pH effective to ensure consumption by the microbial broth of the stream B to generate a biomass product and a liquid vehicle. Bioreactor 12 is supplied with air (oxygen) through line 207. The one or more bio-products and a liquid vehicle are supplied on in line 209 to biomass separator 13, and vent gas leaves the bioreactor through line 208. The biomass separator 13 separates the one or more bio-products, which is removed through line 210, and a recycle stream A which is recycled to gas contactor 11 in line 203 after combination with make-up water in line 204.

The fluid feedstock stream supplied in line 201 is (in this example) flue gas from a chemical plant - but other sources such as power plant or other industrial process could be used.

The feedstock gas is supplied in line 201 at atmospheric pressure to a pressure booster 10 which increases the pressure of the feedstock gas to an extent so the flue gas overcomes hydraulic head in gas contactor 11. Gas contactor 11 is supplied in line 203 with a liquid recycle stream from the process, in combination with make-up water from line 204.

In gas contactor 11 carbon dioxide and H₂S in the feedstock stream are dissolved in the alkaline liquid recycle stream and supplied on in line 205 to bioreactor 12. A substantially carbon dioxide-free and hydrogen sulfide-free off-gas is vented in line 206.

Stream B in line 205 enters bioreactor 12 charged with a microbial broth and maintained under conditions of temperature/pressure/pH effective to ensure consumption by the microbial broth of the stream B to generate one or more bio-products and a liquid vehicle. Both are supplied on in line 207 to biomass separator 13, from which is recovered in line one or more bio-products and, in line 208, a recycle stream A which is recycled to gas contactor 11 in line 203 after combination with make-up water in line 204.

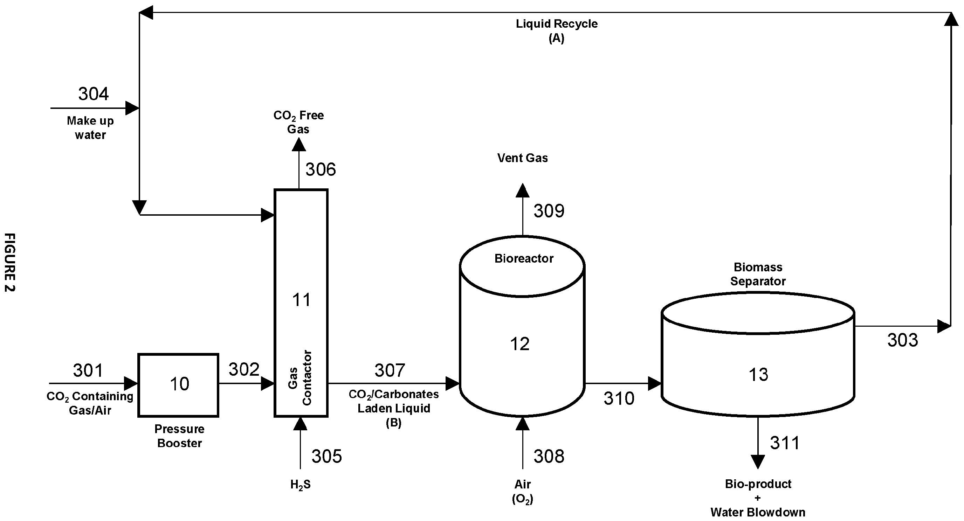

### Figure 2 and Example

In the process of the invention schematically represented by Figure 2 the feedstock gas comprising CO₂ is supplied in line 301 at atmospheric pressure to a pressure booster 10 which increases the pressure of the feedstock gas to an extent so the flue gas overcomes hydraulic head in gas contactor 11. Pressurized gas passes through line 302 to gas contactor 11, which is also supplied by line 303 with a liquid recycle stream from the process, in combination with make-up water from line 304. The gas contactor is also supplied with a separate stream containing H₂S from line 305. In gas contactor 11, CO₂ and H₂S are dissolved in the liquid recycle stream and is supplied on in line 307 to bioreactor 12. A substantially carbon dioxide free and hydrogen sulfide free off-gas is vented in line 306.

The fluid feedstock stream supplied in line 301 is (in this example) flue gas from a chemical plant - but other sources such as power plant or other industrial process could be used.

The fluid feedstock stream supplied in line 305 is (in this example) an H₂S stream - but other sources such as chemical or power plant or other industrial process could be used.

Stream B in line 307 enters bioreactor 12 charged with a microbial broth and maintained under conditions of temperature/pressure/pH effective to ensure consumption by the microbial broth of the stream B to generate a biomass product and a liquid vehicle. Bioreactor 12 is supplied with air (oxygen) through line 308. The one or more bio-products and a liquid vehicle are supplied on in line 310 to biomass separator 13, and vent gas leaves the bioreactor through line 309. The biomass separator 13 separates the one or more bio-products, which is removed through line 311, and a recycle stream A which is recycled to gas contactor 11 in line 303 after combination with make-up water in line 304.

### EXAMPLE

In the process of the invention schematically represented in Figure 2 a carbon containing feedstock stream (flue gas) and an H2S feedstock stream (H2S gas) are supplied to a Gas Contactor 11.

The composition of the Flue gas is as follows:

| **Material** | **%v/v** |
|---|---|
| N2 | 75 |
| CO2 | 10 |
| H2O | 9 |
| O2 | 5 |
| SO2 | 300 (ppm) |
| CO | 700 (ppm) |
| NOX | 150 (ppm) |
| SO3 | 20 (ppm) |

The flue gas may be supplied at atmospheric pressure or elevated pressure. If supplied at atmospheric pressure, a pressure booster may be used to increase pressure of the flue gas to overcome hydraulic head in gas contactor 11. Gas contactor 11 is also supplied by H2S containing stream. Gas contactor 11 is also supplied a liquid recycle stream from the process. In gas contactor 11 carbon dioxide and hydrogen sulfide are dissolved into the liquid stream and supplied to bioreactor 12. A substantially carbon dioxide free and hydrogen sulfide free off gas is vented as off gas.

The composition of the H2S containing stream is as follows:

| Material | %v/v |
|---|---|
| H2S | 100% |

The composition of the liquid stream fed into bioreactor 12 is as follows:

| Material | Concentration (Ibmol/hr) |
|---|---|
| CO₂ | 0.47 |
| OH⁻ | 2.89 |
| HCO₃⁻ | 1419.50 |
| CO₃²⁻ | 1043.09 |
| HS⁻ | 476.3 |

The composition of the off gas vented from gas contactor 11 is as follows:

| Material | %v/v |
|---|---|
| N2 | 85 |
| H2O | 9 |
| O2 | 5 |
| Trace components | 1% |

Bioreactor 12 is fed with the liquid stream from gas contactor 11 and with compressed air as an oxygen source.

Bioreactor 12 is charged with a microbial broth and maintained under conditions of temperature/pressure/pH effective to ensure consumption by the microbial broth of the liquid stream fed from gas contactor 11 to generate a sulfur-containing biomass product and a liquid vehicle. The sulfur-containing biomass and the liquid vehicle are supplied to Biomass Separator 13. A concentrated sulfur-containing biomass and a substantially biomass-free liquid are produced. The biomass-free liquid is returned to the gas contactor 11 via line 303. In some embodiments, the concentrated sulfur-containing biomass can be processed further to separate elemental sulfur from the biomass. In other embodiments, the concentrated sulfur-containing biomass is used directly as a feedstock for further bioreactors.

In certain embodiments, the bioreactor 12 operating parameters are optimized to promote biomass growth. In other embodiments, the bioreactor operating parameters are optimized to promote lipid buildup. In still more embodiments, the bioreactor operating parameters are optimized to promote carbohydrate accumulation

The above described flue gas and H2S containing gas stream can be processed according to the invention substantially to remove the hydrogen sulfide and capture the carbon dioxide.

### Figure 3

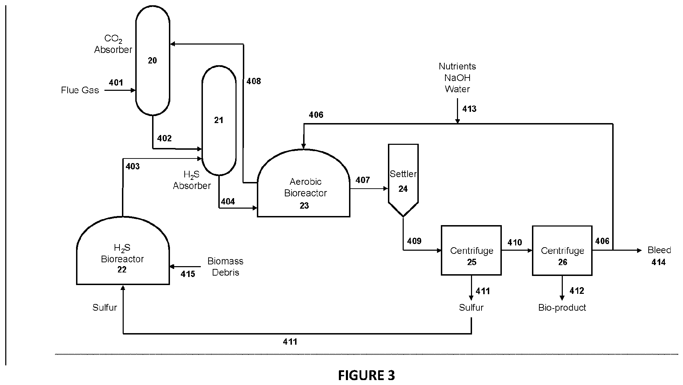

In the process of the invention schematically represented by Figure 3 elemental sulfur product from the process of the invention is recycled via an H₂S bioreactor to provide a sulfurous stream for the process of the invention.

Flue gas is supplied to CO₂ absorber 20 through line 401. In CO₂ absorber 20 the carbon dioxide is absorbed by a recycled liquid stream, passing through line 402 to a H₂S absorber 21. H₂S absorber 21 is also supplied by a stream 403 from H₂S bioreactor 22. In H₂S absorber 21, the hydrogen sulfide is absorbed into the recycled liquid stream.

The fluid feedstock stream supplied in line 401 is (in this example) flue gas from a chemical plant - but other sources such as power plant or other industrial process could be used.

The feedstock gas may be supplied at atmospheric pressure or elevated pressure. If supplied at atmospheric pressure, a pressure booster may be used to increase pressure of the sour gas to overcome hydraulic head in gas absorber 20.

The carbon and sulfur containing reagent stream passes from through line 404 to aerobic bioreactor 23 charged with a microbial broth and maintained under conditions of temperature/pressure/pH effective to ensure consumption by the microbial broth of the stream to generate one or more bio-products and a liquid vehicle. Bioreactor 23 is supplied with nutrients, sodium hydroxide and water through line 406, which also contains recycled liquid vehicle. The one or more bio-products and a liquid vehicle are supplied on in line 407 to settler 24, from which is recovered one or more bio-products. In line 408, a recycle stream of CO₂ is recycled to CO₂ absorber 20.

The mixture of one or more bio-products and a liquid vehicle pass from settler 24 through line 409, firstly to centrifuge 25, which is operated under conditions to separate elemental sulfur through line 411. The substantially sulfur-free product mixture passes from centrifuge 25, through line 410, to centrifuge 26, which operates under conditions to separate the bio-products from the liquid vehicle. The desired bioproduct is collected through line 412 and the recycled liquid vehicle is removed through line 406 and recycled to the aerobic bioreactor, being combined with nutrients, sodium hydroxide and water supplied through line 413. A bleed outlet 414 is also present to allow the removal of excess liquid vehicle.

The sulfur removed through line 411 is provided to H₂S bioreactor 22, which is also supplied with biomass debris from line 415. H₂S bioreactor is charged with sulfate reducing bacteria producing a stream of H₂S that is fed back into the H₂S absorber 21 through line 403 to improve carbon capture.

For the avoidance of doubt, all features relating to process for sequestering carbon dioxide also relate, where appropriate to the apparatus for sequestering carbon dioxide and *vice versa.*

## Claims

1. A microbiological process for the conversion of CO₂ into one or more bio-products, in which the energy required for the microbiological conversion is at least partially provided by the concomitant microbiological oxidation of a sulfurous feedstock.

2. The microbiological process according to claim 1 effected by one or more chemotrophic organisms, optionally wherein one or more of the chemotrophic organisms are sulfur oxidising bacteria.

3. The process according to claim 1 or claim 2 wherein the microbiological conversion and/or the concomitant microbiological oxidation is effected by one or more organisms selected from Thioalkalimicrobium, Thioalkalivibrio, Thiobacillus, Alkalilimnicola, Guyparkeria, Halomonas, Alkalispirillum, Vibrio, Thiomicrospira, Guyparkeria, Thioalkalispira (formerly Thioalkalimicrobium), Ectothiorhodospiraceae, Rhodobacteraceae, Roseinatrobacter, Alkalilimnicola, Guyparkeria, Desulfuromusa, Desulfurispirillum.

4. The microbiological process according to any one of claims 1 to 3 wherein the sulfurous feedstock is at least partially oxidized to elemental sulfur by sulfur oxidising bacteria.

5. The process according to any one of claims 1 to 4 wherein the reduced elemental sulfur is removed from the one or more bio-products by a method selected from centrifugation, solvent extraction, or by means of a hydrocyclone.

6. The microbiological process according to any one of claims 1 to 5 comprising the steps of:
i. contacting a raw carbon dioxide and sulfur-containing feedstock with an absorption or dissolution medium to form a reagent stream comprising dissolved or absorbed inorganic carbon and sulfur; and
ii. contacting at least a portion of the reagent stream with a microbial broth in a bioreactor to oxidize sulfur and produce one or more bio-products from the carbon dioxide; optionally wherein the process further comprises:
iii. separating the one or more bio-products into one or more bio-products and a liquid stream; and
iv. recycling at least a portion of the liquid stream to step i. of the process for use as, or part of, the absorption or dissolution medium.

7. The process according to claim 6 wherein either:
i. carbon dioxide and the sulfur are introduced into the process as or as part of a single feedstock stream; or
ii. carbon dioxide and the sulfur are introduced into the process as separate feedstock streams.

8. The process according to claim 6 or claim 7 wherein:
i. in-line monitoring is used to determine the carbon dioxide/sulfur ratio and/or carbon speciation;
ii. the pH of the absorption or dissolution medium is controlled; and/or
iii. the process is operated in a nutrient-starved environment and the one or more bio-products is lipid-rich.

9. The process according to any one of claims 1 to 8 wherein:
i. debris from the one or more bio-products is used as a feedstock for an anaerobic digester to generate biogas; and/or
ii. debris from the one or more bio-products is used as a feedstock for a secondary bioreactor, wherein the reactor uses sulphate reducing bacteria to produce H₂S, optionally wherein the H₂S produced by the secondary bioreactor is used as or as part of the sulfurous feedstock, optionally wherein the secondary bioreactor produces biologic chemicals.

10. The process according to any one of claim 1 to 9, wherein the one or more bio-products comprises biomass, lipids for fuels, PHAs or BDO.

11. An apparatus for the conversion of CO₂ into one or more bio-products, in which the energy required for the microbiological conversion is at least partially provided by the concomitant microbiological oxidation of a sulfurous feedstock, the apparatus comprising:
i. means for contacting raw carbon dioxide and sulfur-containing feedstocks, either separately or in combination, with an absorption or dissolution medium to form a reagent stream comprising dissolved or absorbed inorganic carbon and sulfur; and
ii. means for contacting at least a portion of the reagent stream with a microbial broth in a bioreactor to oxidize sulfur and produce one or more bio-products from the carbon dioxide.

12. An apparatus according to claim 11 further comprising a gas contactor to concentrate the fluid stream.

13. The apparatus according to claim 11 or claim 12, further comprising a secondary bioreactor charged with sulphate reducing bacteria.

14. An apparatus according to any of claims 11 to 13 configured to operate the process of any one of claims 1 to 10.

15. One or more bio-products produced by a process according to any one of Claims 1 to 10.
